# EUROPÄISCHE PATENTSCHRIFT

(11) **EP 2 446 923 B1**
(45) Veröffentlichungstag und Bekanntmachung des Hinweises auf die Patenterteilung: **06.12.2017**
(21) Anmeldenummer: 11184601.0
(22) Anmeldetag: 11.10.2011
(51) Int. Cl.: A61N 1/16, G01R 33/422, A41D 13/008, A41D 13/12, A41D 31/00, G12B 17/02, H05K 9/00, A61F 7/00, A61N 1/37, A61N 1/04

(54) **ABSCHIRMVORRICHTUNG FÜR MAGNETRESONANZTOMOGRAPHIE**
SHIELDING APPARATUS FOR MAGNETIC RESONANCE IMAGING
DISPOSITIF DE BLINDAGE POUR TOMOGRAPHIE PAR RÉSONANCE MAGNÉTIQUE

(30) Priorität: 28.10.2010 US 407479 P
(43) Veröffentlichungstag der Anmeldung: 02.05.2012
(73) Patentinhaber: BIOTRONIK SE & Co. KG, 12359 Berlin (DE)
(72) Erfinder: DÖRR, Thomas, 12437 Berlin (DE); WEISS, Ingo, 12435 Berlin (DE)
(74) Vertreter: Galander, Marcus

(56) Entgegenhaltungen:
- EP-A1- 1 205 589
- WO-A1-01/37286
- DE-U1- 20 113 511
- JP-A- 9 310 208
- US-A1- 2008 023 010

## Beschreibung

Die Erfindung liegt auf dem Gebiet der Medizintechnik und betrifft eine Abschirmvorrichtung für die Magnetresonanztomographie, die geeignet ist zum Einsatz in einem Magnetresonanztomographen.

Bei der Kernspin- oder Magnetresonanztomographie (MRT = Magnetresonanztomographie) werden Atomkerne durch starke statische Magnetfelder zu einer Präzessionsbewegung um die Feldrichtung gezwungen und durch hochfrequente elektromagnetische Wechselfelder resonant angeregt. Nach Abschalten der Wechselfelder kehren die Atomkerne wieder in ihre Ausgangsrichtung zurück, wobei die Abklinkzeiten (Relaxationszeiten) charakteristisch für verschiedene Gewebsarten sind, so dass eine genaue Bildgebung ermöglicht ist.

MRT-Untersuchungen gehören mittlerweile zur täglichen Routine in der radiologischen Diagnostik, wobei unter normalen Untersuchungsbedingungen, abgesehen von gelegentlichen Befindlichkeitsstörungen, keine Gefährdung für die Patienten besteht. Allerdings besitzen immer mehr Patienten ganz oder teilweise aus einem metallischen Material bestehende Implantate, die Probleme bei der Untersuchung verursachen und die Patienten im schlimmsten Falle sogar verletzen können. Eine Rolle hierbei spielt das verwendete Material und die Geometrie des Implantats, sowie dessen Lage in Bezug auf die eingesetzten hochfrequenten Wechselfelder. So wirken manche Implantate besonders gut als Antenne für die Wechselfelder und erwärmen sich durch die dabei erzeugten Induktionsströme stark. In der Folge kann eine thermische Schädigung des umliegenden Gewebes auftreten. Andererseits kann in elektronischen Implantaten durch die hochfrequenten Wechselfelder eine Fehlfunktion oder ein unerwünschter Neustart mit voreingestellten Startparametern ("power-on reset") ausgelöst werden.

Aus diesem Grund muss der diagnostische Nutzen einer magnetresonanztomographischen Untersuchung gegenüber dem Risiko nachteiliger Nebenwirkungen sorgfältig abgewogen werden. Erschwerend kommt hinzu, dass beispielsweise die Erwärmung implantierter Elektroden von den jeweiligen Scan-Parametern der Untersuchung und insbesondere von deren Positionierung im elektromagnetischen Wechselfeld abhängt, so dass in der klinischen Praxis oftmals keine verlässlichen Vorhersagen über die zu erwartenden Auswirkungen getroffen werden können. All dies führt dazu, dass Patienten mit einem Implantat in der Regel von der an sich schonenden Untersuchung mittels Magnetresonanztomographie ausgeschlossen sind.

In jüngster Zeit wurden vermehrt Anstrengungen unternommen, dieses Problem zu lösen. So wurden beispielsweise neue Herzschrittmachersysteme entwickelt, die über geometrisch speziell gestaltete Schrittmacherelektroden verfügen, um einer Erwärmung durch die elektromagnetischen Wechselfelder entgegen zu wirken. Nachteilig hierbei ist, dass diese Lösungen mit einem zusätzlichen Aufwand für die Implantate und gegebenenfalls auch mit einer Beeinträchtigung der Implantateigenschaften verbunden sind. Darüber hinaus können Patienten, die bereits ein Implantat besitzen, weiterhin nicht mittels Magnetresonanztomographie untersucht werden.

Die US-Patentanmeldung US 2008/0023010 A1 beschreibt Verfahren und Vorrichtungen zur Reduktion der durch RF-Felder induzierten Erwärmung von Implantaten. Es wird eine RF-Abschirmung gezeigt, die eine lokale Abschirmung eines Implantats erlaubt, während gleichzeitig eine Exposition von anderen Körperteilen eines Patienten möglich ist. Die beschriebene RF-Abschirmung besteht aus einer energieabsorbierenden Struktur die um den Patienten gewickelt ist. Die energieabsorbierende Struktur kann aus Karbonfasern, aus leitfähigen metallfasern oder aus Kombinationen hiervon bestehen. Ein Vorteil dieser RF-Abschirmung ist, dass sie nicht implantiert zu werden braucht.

Das deutsche Gebrauchsmuster DE 20 113 511 U1 zeigt ein Unterhemd, mit Abschirmung vor elektromagnetischen Wellen für Herzschrittmacher-Träger, das aus Hochfrequenzabschirmendem Baumwollgewebe gefertigt ist, in das haarfeine Silberfäden eingewoben sind.

Die PCT-Anmeldung WO 01/37286 A1 beschäftigt sich mit Kleidung, die elektromagnetische Felder abschirmt. Diese Kleidung kann im täglichen Leben von einer Person getragen werden, um diese Person vor in der Umwelt auftretenden elektromagnetischen Feldern, die von elektronischen Geräten des täglichen Lebens verursacht werden, zu schützen. Die elektromagnetische Felder abschirmende Kleidung kann weiterhin bei Personen angewendet werden die elektronische Implantate tragen, bei denen es durch die von in der Umwelt auftretenden elektromagnetischen Feldern zu Störungen kommen kann.

Die europäische Patentanmeldung EP 1 205 589 A1 bezieht sich auf Kleidung und zusätzlich auf ein reversibel gestricktes Material zur Abschirmung von elektromagnetischen Feldern.

Auch das Japanische Patentdokument JP 09310208 A beschäftigt sich mit Kleidung zur Abschirmung elektromagnetischer Wellen aus elektrisch leitfähigem Stoff.

Demgegenüber besteht die Aufgabe der vorliegenden Erfindung darin, es zu ermöglichen, Patienten mit einem Implantat gefährdungslos einer magnetresonanztomographischen Untersuchung unterziehen zu können, ohne zu diesem Zweck spezielle Vorkehrungen am Implantat treffen zu müssen. Diese und weitere Aufgaben werden nach dem Vorschlag der Erfindung durch eine Abschirmvorrichtung und Abschirmanordnungen mit den Merkmalen des unabhängigen Patentanspruchs gelöst. Vorteilhafte Ausgestaltungen der Erfindung sind durch die Merkmale der Unteransprüche angegeben.

Erfindungsgemäß ist eine Abschirmvorrichtung zur medizinischen Anwendung gezeigt, welche zur Abschirmung wenigstens eines zumindest teilweise aus einem metallischen Material bestehenden Implantats im Körper eines Patienten bei einer magnetresonanztomographischen Untersuchung (MRT-Untersuchung) dient. Wie hier verwendet, bezeichnet der Ausdruck "Implantat" eine für einen dauerhaften oder nur vorübergehenden Verbleib im Körper eines Patienten vorgesehene Vorrichtung, bei der es sich beispielsweise um ein elektronisches Gerät, wie einen Herzschrittmacher oder Kardioverter, eine Elektrode, wie eine Stimulationselektrode, insbesondere zur Nervenstimulation, eine Herzschrittmacherelektrode, eine ICD-Elektrode, einen Elektrophysiologie-Katheter oder eine Elektrode zur Messung von Hirnpotentialen oder dergleichen handeln kann. Es versteht sich, dass diese Aufzählung nicht abschließend ist.

Die erfindungsgemäße Abschirmvorrichtung ist so ausgebildet, dass sie vorübergehend am oder nahe dem Körper des Patienten anbringbar ist und ein Abschirmmaterial umfasst, das geeignet ist, bei der MRT-Untersuchung genutzte, hochfrequente, elektromagnetische Wechselfelder zu dämpfen. In der Regel handelt es sich dabei um elektromagnetische Wechselfelder, deren Frequenz in einem Frequenzbereich oberhalb von 1 MHz liegt. Beispielsweise können Protonen bei einem statischen Magnetfeld von 1 Tesla mit einem elektromagnetischen Wechselfeld einer Frequenz von ca. 42 MHz, bei einem statischen Magnetfeld von 1,5 Tesla mit einem elektromagnetischen Wechselfeld einer Frequenz von ca. 63 MHz angeregt werden. Das Abschirmmaterial ist so ausgebildet, dass eine nennenswerte Dämpfungswirkung für die bei der MRT-Untersuchung genutzten, hochfrequenten Wechselfelder erzielt wird, die jedenfalls so stark ist, dass eine mit einer möglichen Gefährdung des Patienten einher gehende Beeinträchtigung des Implantats verhindert wird. So soll eine Fehlfunktion eines elektronischen Implantats verhindert und die Erwärmung einer Elektrode stark reduziert bzw. minimiert werden. Ohne hierauf eingeschränkt zu sein, kann durch das Abschirmmaterial beispielsweise eine Dämpfungswirkung erzielt werden, durch welche die Intensität, bezogen auf die Intensität der auf die Abschirmvorrichtung treffenden Wechselfelder, so reduziert wird, dass sie kleiner als 50%, insbesondere kleiner als 40%, insbesondere kleiner als 30%, insbesondere kleiner als 20%, insbesondere kleiner als 10%, insbesondere kleiner als 5% und insbesondere kleiner als 1%, ist.

Um für die hochfrequenten Wechselfelder dämpfend wirken zu können, d. h. um die Intensität der Wechselfelder zu reduzieren, ist das Abschirmmaterial elektrisch leitfähig. Allgemein kann das Abschirmmaterial in Form eines elektrisch leitfähigen Festkörpers und/oder einer elektrisch leitfähigen Flüssigkeit vorliegen, wobei es beispielsweise als Granulat und/oder Polymermaterial und/oder in Form von Kohlefasern ausgebildet sein kann. Insbesondere kann das Abschirmmaterial beispielsweise als Netz aus Fäden eines elektrisch leitfähigen Materials oder als elektrisch leitfähige Folie vorliegen.

Um die Abschirmvorrichtung vorübergehend am oder nahe dem Körper des Patienten anbringen zu können, ist sie vorteilhaft in Form einer Weste, Hose, Hosenbein, Halskrause, Kappe, Kopfmaske mit Öffnungen für Augen und Atemöffnungen oder als ein um den Körper oder ein Körperteil des Patienten wickelbares Teil ausgebildet. Als Weste dient die Abschirmvorrichtung insbesondere zur Abschirmung eines Implantats im thorakalen Bereich, beispielsweise ein Herzschrittmacher. Als Hose oder Hosenbein dient sie insbesondere zur Abschirmung einer langgestreckten metallischen Gefäßprothese bzw. Stent, die häufig eine Länge von wenigstens 200 mm aufweisen. Als Kappe bzw. Kopfmaske dient sie insbesondere zur Abschirmung eines so genannten Deep Brain-Stimulators.

Die Abschirmvorrichtung kann für eine Befestigung am Patienten beispielsweise mit Verschlüssen wie Klettverschlüssen versehen sein, die vorzugsweise aus einem elektrisch leitfähigen Material ausgebildet sind, um eine vollständige Abschirmung zu gewährleisten. Denkbar ist auch, die Abschirmvorrichtung mit einer oder mehreren Klebstellen zu versehen, so dass sie zu deren Fixierung auf eine Körperoberfläche des Patienten aufklebbar ist. Die Abschirmvorrichtung kann zumindest teilweise aus atmungsaktiven Materialien bestehen, um den Komfort für den Patienten zu verbessern. Allgemein kann die Abschirmvorrichtung zum ein- und/oder mehrmaligen Gebrauch vorgesehen sein.

Die erfindungsgemäße Abschirmvorrichtung ermöglicht somit in vorteilhafter Weise eine einfache, kostengünstige und für den Patienten gefährdungsfreie MRT-Untersuchung, ohne dass spezielle Vorkehrungen am Implantat zu treffen sind. Insbesondere können mithilfe der erfindungsgemäßen Abschirmvorrichtung auch Patienten, die bereits ein Implantat besitzen, einer MRT-Untersuchung unterzogen werden. Beispielsweise können auch Patienten mit einer defekten und/oder einer stillgelegten Elektrode oder Sonde einer MRT-Untersuchung zugeführt werden. Andererseits kann die erfindungsgemäße Abschirmvorrichtung auch zum Schutz von medizinischem Personal mit einem zumindest teilweise aus Metall bestehenden Implantat verwendet werden.

Die erfindungsgemäße Abschirmvorrichtung ist für die in der Magnetresonanztomographie genutzten hochfrequenten elektromagnetischen Wechselfelder dämpfend wirksam. Bei einer vorteilhaften Ausführungsform der erfindungsgemäßen Abschirmvorrichtung weist sie zudem eine relative magnetische Permeabilität von maximal 100, insbesondere von maximal 4, insbesondere von maximal 1 auf. Dabei kann es von Vorteil sein, wenn die Abschirmvorrichtung nur aus para- und/oder diamagnetischen bzw. magnetisch neutralen Materialien besteht. Durch diese Maßnahme kann erreicht werden, dass die Abschirmvorrichtung für die in der MRT-Untersuchung verwendeten starken statischen Magnetfelder sowie für die dabei eingesetzten magnetischen Gradientenfelder transparent ist, um eine Verzerrung dieser Felder zur Verminderung bzw. Verhinderung von Artefakten zu vermeiden. Zudem kann in vorteilhafter Weise eine Bildgebung nahe der Abschirmvorrichtung erfolgen.

Die erfindungsgemäße Abschirmvorrichtung ist geeignet, das Implantat des Patienten von den bei der MRT-Untersuchung eingesetzten elektromagnetischen Wechselfeldern zumindest teilweise, insbesondere (praktisch) vollständig abzuschirmen. Dies wird durch die dämpfende Wirkung des Abschirmmaterials erreicht, wobei die Dämpfungswirkung des Abschirmmaterials vorzugsweise besonders hoch für die bei der MRT-Untersuchung genutzten elektromagnetischen Wechselfelder ist. Insbesondere ist möglich, dass das Abschirmmaterial eine frequenzabhängige Dämpfungswirkung aufweist, welche an die hochfrequenten elektromagnetischen Wechselfelder der MRT-Untersuchung bzw. an einen bestimmten Scanner-Typ (Magnetresonanztomographen) angepasst ist.

Bei einer weiteren vorteilhaften Ausführungsform der erfindungsgemäßen Abschirmvorrichtung ist eine physikalische und/oder chemische Eigenschaft des Abschirmmaterials zur Änderung seines Dämpfungsvermögens zur Dämpfung der in der MRT-Untersuchung genutzten elektromagnetischen Wechselfelder veränderbar. Zu diesem Zweck kann es von Vorteil sein, wenn die Abschirmvorrichtung so ausgebildet ist, dass die Menge des Abschirmmaterials bzw. das Volumen eines flüssigen Abschirmmaterials veränderbar ist. Insbesondere kann es von Vorteil sein, wenn das Abschirmmaterial von der Abschirmvorrichtung teilweise oder vollständig entfernbar ist, so dass das Abschirmmaterial zumindest teilweise ausgetauscht werden kann. Auf diese Weise kann die Dämpfungswirkung in Abhängigkeit der Patienten und/oder der spezifischen Scan-Parameter und/oder des eingesetzten Scannertyps gezielt eingestellt werden. Zudem kann durch eine Mengen- bzw. Volumenänderung des Abschirmmaterials eine Dicke (d. h. Abmessung senkrecht zur Körperoberfläche des Patienten) der Abschirmvorrichtung bedarfsgerecht eingestellt werden. Weiterhin kann es von Vorteil sein, wenn die Abschirmvorrichtung so ausgebildet ist, dass zum Zwecke einer Änderung der Dämpfungswirkung die elektrische Leitfähigkeit des Abschirmmaterials veränderbar ist. Bei einem flüssigen Abschirmmaterial kann eine Änderung der elektrischen Leitfähigkeit beispielsweise durch den variablen Gehalt einer ionisch gelösten Substanz verändert werden. Allgemein kann die Dämpfungswirkung der Abschirmvorrichtung durch eine Mengenvariation und/oder Änderung der Zusammensetzung und/oder Austausch des Abschirmmaterials gezielt auf die Bedürfnisse der Nutzer eingestellt werden.

Die erfindungsgemäße Abschirmvorrichtung ist zur Anpassung an eine Kontur einer Körperoberfläche des Patienten geeignet ausgebildet. Durch diese Maßnahme kann einerseits eine besonders gute Abschirmung des Implantats von den bei der MRT-Untersuchung eingesetzten Wechselfeldern erreicht werden. Andererseits erhöht sich hierdurch der Komfort für den Patienten. Die Abschirmvorrichtung kann zu diesem Zweck beispielsweise aus flexiblen bzw. biegsamen Materialien gefertigt sein. Vorzugsweise ist die Abschirmvorrichtung dabei so ausgebildet, dass ein (kürzester) Abstand zwischen der Abschirmvorrichtung und der Körperoberfläche des Patienten an jeder Stelle nicht größer als 5 cm, stärker bevorzugt nicht größer als 3 cm und noch stärker bevorzugt nicht größer als 1 cm ist. Durch diese Maßnahme kann ein besonders guter abschirmender Effekt gegen die hochfrequenten Wechselfelder erzielt werden. Bei einer Anwendung der Abschirmvorrichtung im thorakalen Bereich ist es bevorzugt, wenn die Flexibilität der Abschirmvorrichtung dergestalt ausgebildet ist, dass die Atmung des Patienten nicht nennenswert beeinträchtigt ist. Zudem kann die Abschirmvorrichtung zumindest teilweise aus atmungsaktiven Materialien bestehen. Die Abschirmvorrichtung ist zur Anpassung an die Kontur der Körperoberfläche des Patienten mit einem gasförmigen und/oder flüssigen Fluid befüllbar. Bei dem flüssigen Fluid kann es sich insbesondere um das Abschirmmaterial handeln.

Bei einer weiteren vorteilhaften Ausführungsform der erfindungsgemäßen Abschirmvorrichtung ist sie aus einer Mehrzahl Segmente aufgebaut, die galvanisch oder kapazitiv miteinander gekoppelt sind. Durch diese Maßnahme kann einerseits eine gute Anpassbarkeit der Abschirmvorrichtung an eine Kontur einer Körperoberfläche des Patienten erreicht werden. Andererseits können die Segmente speziell angeordnet werden, um als Resonator für die bei der MRT-Untersuchung eingesetzten hochfrequenten Wechselfelder zu wirken und somit die Abschirmwirkung für das Implantat zu verbessern.

In einer besonders vorteilhaften Ausführungsform wird durch eine geeignete Dimensionierung der Segmente neben der guten Anpassbarkeit der Abschirmvorrichtung an eine Kontur einer Körperoberfläche des Patienten und der Resonatoreigenschaften für die bei der MRT-Untersuchung eingesetzten hochfrequenten Wechselfelder auch erreicht, dass gleichzeitig die Gradientenfelder durch die Abschirmvorrichtung gelassen werden.

Besonders bevorzugt wird auch, dass bei einer kapazitiven Kopplung der Segmente die Kapazität zwischen den Segmenten zwischen 100pF pro cm² und 10nF pro cm² beträgt.

Die erfindungsgemäße Abschirmvorrichtung kann sich aufgrund von durch die hochfrequenten Wechselfelder erzeugten Induktionsströme erwärmen. Vorzugsweise weist die Abschirmvorrichtung eine Kühleinrichtung zu der aktiven oder passiven Kühlung auf. Durch diese Maßnahme kann in vorteilhafter Weise einerseits der Komfort für den Patienten bei der Anwendung der Abschirmvorrichtung verbessert werden. Andererseits kann die Zeitdauer der MRT-Untersuchung und gegebenenfalls die Intensität der bei der Untersuchung eingesetzten hochfrequenten Wechselfelder erhöht werden. Die Kühleinrichtung kann insbesondere als Vorrichtung zur aktiven Belüftung ausgebildet sein. Denkbar wäre auch, für eine aktive Kühlung auf dem Peltier-Effekt basierende Peltier-Elemente einzusetzen, welche je nach Polung der angelegten Spannung Wärme erzeugen oder abführen. Eine passive Kühlung kann beispielsweise durch vorgekühlte Kühlakkus erreicht werden.

Bei einer weiteren vorteilhaften Ausführungsform der erfindungsgemäßen Abschirmvorrichtung ist die Abschirmvorrichtung mit einer Kommunikationseinrichtung zur Kommunikation mit dem (elektronischen) Implantat ausgerüstet. Auf diese Weise können dem Implantat Informationen übermittelt werden, wobei insbesondere die Information übermittelbar ist, dass die erfindungsgemäße Abschirmvorrichtung verwendet und eine MRT-Untersuchung durchgeführt wird. Das Implantat kann beispielsweise so programmiert sein, das es daraufhin in einen MRT-Betriebsmodus schaltet, der zur Vermeidung von Fehlfunktionen an die MRT-Untersuchung speziell angepasst ist.

Vorzugsweise ist die Kommunikationseinrichtung der Abschirmvorrichtung dabei so ausgebildet, dass sie durch Energie aus dem hochfrequenten Wechselfeld des Magnetresonanztomographen gespeist werden kann, so dass einerseits keine separate Energieversorgung für die Kommunikationseinrichtung erforderlich ist und andererseits nur dann arbeitet, wenn die MRT-Untersuchung tatsächlich durchgeführt wird. Die Aktivität der Kommunikationseinrichtung wird somit durch die Energie der hochfrequenten Wechselfelder gesteuert, wobei das Implantat bei fehlender Aktivität der Kommunikationseinrichtung die Information erhält, dass die Bildgebung bzw. das Erzeugen hochfrequenter Wechselfelder beendet ist und somit wieder in den normalen Betriebsmodus schalten kann.

Bei einer weiteren vorteilhaften Ausführungsform der erfindungsgemäßen Abschirmvorrichtung weist sie eine Markierungseinrichtung zu ihrer Markierung in einer bei der Untersuchung erstellten magnetresonanztomographischen Aufnahme auf. Durch diese Maßnahme kann in vorteilhafter Weise eine automatische Bildkorrektur bzw. Artefaktabgrenzung erfolgen, um die Qualität der Bilderzeugung zu verbessern. Die Markierungseinrichtung kann insbesondere als so genannter RFID-Transponder (RFID = Radio-frequency identification) ausgebildet sein, der eine automatische Identifizierung und Markierung mittels elektromagnetischer Wellen ermöglicht.

Bei einer weiteren vorteilhaften Ausführungsform der erfindungsgemäßen Abschirmvorrichtung ist diese so ausgebildet, dass sich deren Dicke (d. h. Abmessung senkrecht zu einer Körperoberfläche des Patienten) bei der Anwendung am Patienten aufgrund einer mechanischen Krafteinwirkung, beispielsweise durch das Gewicht des Patienten, wenn überhaupt, nur geringfügig ändert. Vorzugsweise erfährt die Abschirmvorrichtung dabei eine Dickenänderung die geringer als 20% ist. Durch diese Maßnahme kann eine zuverlässige und sichere Dämpfungswirkung der Abschirmvorrichtung sichergestellt werden. Dies gilt insbesondere für Abschirmvorrichtungen, die mit einem flüssigen Abschirmmaterial befüllbar sind. Die Abschirmvorrichtung kann zu diesem Zweck beispielsweise mit einer Wabenstruktur versehen sein.

Bei einer weiteren vorteilhaften Ausführungsform der erfindungsgemäßen Abschirmvorrichtung ist ein Randbereich (Saum) der Abschirmvorrichtung so ausgebildet, dass eine übermäßige Erwärmung des Körpergewebes des Patienten durch einen lokal erhöhten spezifischen Absorptionswert (SAR) vermieden wird. Dies kann beispielsweise dadurch erreicht werden, dass die Abschirmvorrichtung so ausgebildet ist, dass deren Abschirmeffizienz bzw. Dämpfungswirkung für die hochfrequenten Wechselfelder zum Rand hin beispielsweise graduell abnimmt und/oder dass ein (kürzester) Abstand zwischen der Abschirmvorrichtung und der Körperoberfläche des Patienten zum Rand hin zunimmt. Durch diese Maßnahme kann insbesondere der Komfort für den Patienten verbessert werden. Andererseits können die Zeitdauer der Untersuchung und gegebenenfalls die Intensität der hochfrequenten Wechselfelder erhöht werden.

Bei einer weiteren vorteilhaften Ausführungsform der erfindungsgemäßen Abschirmvorrichtung ist ein Randbereich (Saum) der Abschirmvorrichtung so ausgebildet, dass sich eine Wellenimpedanz der hochfrequenten Wechselfelder vom nicht-abgeschirmten Körpergewebe zu dem von der Abschirmvorrichtung abgeschirmten Bereich hin nur geringfügig ändert. Vorzugsweise ändert sich die Wellenimpedanz um weniger als 20%, besonders bevorzugt um weniger als 10% und noch stärker bevorzugt um weniger als 5%. Besonders bevorzugt findet eine Transformation bzw. Anpassung des Wellenwiderstands an den abgeschirmten Bereich innerhalb der ersten 2 bis 5 cm, ausgehend vom Randbereich (Saum) der Abschirmvorrichtung, statt. Durch diese Maßnahme kann in vorteilhafter Weise die Qualität der Bildgebung verbessert werden.

Bei einer weiteren vorteilhaften Ausführungsform der erfindungsgemäßen Abschirmvorrichtung ist diese zur Anwendung im thorakalen Bereich mit Elektrodenflächen versehen, die bei in Stellung gebrachter Abschirmvorrichtung in einen elektrischen Kontakt mit der Körperoberfläche gelangen. Die Elektrodenflächen können insbesondere zur Defibrillation verwendet werden. Vorzugsweise sind die Elektrodenflächen mit Zuleitungen derart verbunden, dass sie von einem externen Defibrillator ohne vorheriges Ablegen der Abschirmvorrichtung angesteuert werden können. Dies bedeutet, dass beispielsweise bei einer westenförmigen Abschirmvorrichtung Anschlüsse oder Auflageflächen zum Anschließen des Defibrillators in vorteilhafter Weise auf der Außenseite der Weste angeordnet sind.

Bei einer weiteren vorteilhaften Ausführungsform der erfindungsgemäßen Abschirmvorrichtung ist diese so ausgebildet, dass sie, wenn sie an oder nahe des Patienten angebracht ist, mit der Körperoberfläche des Patienten galvanisch oder kapazitiv koppelbar bzw. gekoppelt ist. Eine solche Koppelung kann beispielsweise über ein elektrisch leitfähiges Gel erfolgen. Im Fall einer kapazitiven Kopplung kann es von Vorteil sein, wenn die Kapazität größer als 1 pF/m² Koppelfläche ist.

Es versteht sich, dass die verschiedenen Ausführungsformen und Ausgestaltungen der erfindungsgemäßen Gegenstände einzeln oder in beliebigen Kombinationen realisiert sein können. Insbesondere sind die vorstehend genannten und nachstehend zu erläuternden Merkmale nicht nur in den angegebenen Kombinationen, sondern auch in anderen Kombinationen oder in Alleinstellung einsetzbar, ohne den Rahmen der vorliegenden Erfindung zu verlassen.

Die Erfindung wird nun anhand von Ausführungsbeispielen näher erläutert, wobei Bezug auf die beigefügten Zeichnungen genommen wird. Gleiche bzw. gleich wirkende Elemente sind mit denselben Bezugszahlen bezeichnet. Es zeigen:
- Fig. 1: ein typischer Temperaturverlauf der Elektrodenspitze einer implantierten Elektroden bei einer MRT-Untersuchung;
- Fig. 2A-2B: schematische Darstellungen eines Patienten mit Implantat ohne Abschirmvorrichtung (Fig. 2A) und mit einer beispielhaften Abschirmvorrichtung (Fig. 2B);
- Fig. 3: eine schematische Darstellung eines Patienten mit Implantat zur Veranschaulichung einer weiteren beispielhaften Abschirmvorrichtung;
- Fig. 4A-4B: schematische Darstellungen zur Veranschaulichung einer beispielhaften Abschirmanordnung;
- Fig. 5A-5B: rechnerische Simulationen zur Veranschaulichung der Wirkung der Abschirmvorrichtung;
- Fig. 6A-6B: weitere rechnerische Simulationen zur Veranschaulichung der Wirkung der Abschirmvorrichtung;
- Fig. 7: den beispielhaften Verlauf des B1-Felds bei einer MRT-Untersuchung mit und ohne Abschirmvorrichtung.

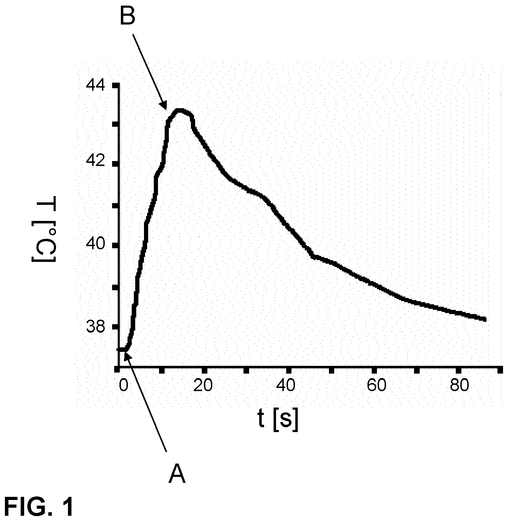

Sei zunächst Fig. 1 betrachtet, worin anhand eines Diagramms ein typischer Temperaturverlauf der Elektrodenspitze einer implantierten konventionellen Schrittmacher/ICD-Elektrode bei einer MRT-Untersuchung dargestellt ist. Als Abszisse ist die Zeit (t) in Sekunden und als Ordinate die Temperatur T in °C aufgetragen. Ursache für die Temperaturerhöhung ist die elektromagnetische Interferenz (Erzeugung von Induktionsströmen). Demnach steigt die Temperatur der Elektrodenspitze mit Einschalten des hochfrequenten Wechselfelds zum Zeitpunkt A rasch an, wobei die Steilheit des Temperaturanstiegs und die maximal erreichbare Temperatur stark von der räumlichen Lage der Elektrode bezüglich des Wechselfelds abhängt. Im gezeigten Beispiel steigt die Temperatur bis über 43°C an. Nach Abschalten des Wechselfelds zum Zeitpunkt B kühlt sich die Elektrodenspitze aufgrund der relativ geringen Wärmekapazität verhältnismäßig schnell wieder ab. Durch die erreichte hohe Temperatur der Elektrodenspitze kann eine thermische Schädigung des umliegenden Gewebes nicht ausgeschlossen werden.

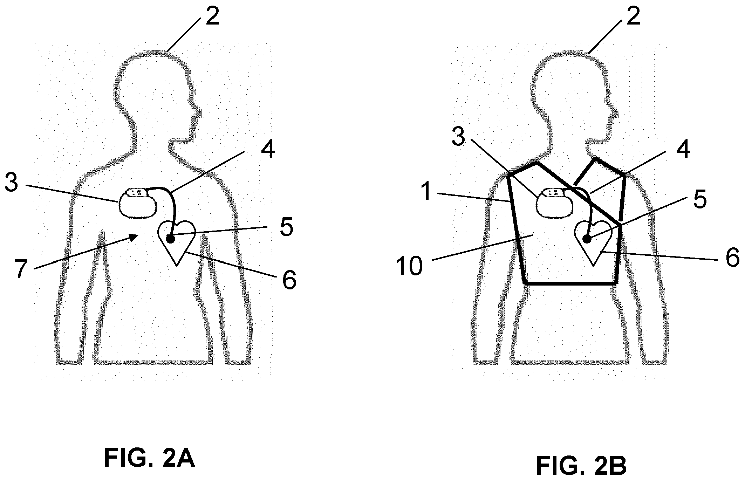

In den Figuren 2A und 2B ist anhand schematischer Darstellungen ein Patient 2 mit einem hier beispielsweise als Schrittmacher 3 ausgeführten elektronischen Implantat gezeigt. Fig. 2A zeigt den Patienten 2 ohne erfindungsgemäße Abschirmvorrichtung 1 und Fig. 2B den Patienten 2 mit angelegter Abschirmvorrichtung 1. Der Schrittmacher 3 ist über eine Elektrodenleitung 4 mit dem Herzen 6 des Patienten verbunden. Die Elektrodenleitung 4 verfügt zu diesem Zweck über einen distalen Abschnitt, welcher beispielsweise den rechten Herzvorhof passiert und mit einem distalen Ende bzw. Elektrodenspitze 5 innerhalb der rechten Herzkammer positioniert ist. Nicht näher dargestellte Elektrodenflächen im Bereich der Elektrodenspitze 5 können für Abtast- und/oder Stimulationszwecke (antibradykarde Stimulation) verwendet werden. Der Schrittmacher 3 kann somit elektrische Signale des Herzens 6 erfassen und elektrische Impulse abgeben, um je nach Bedarf Kontraktionen des Herzens 6 auszulösen. Anstelle eines Schrittmachers 3 könnte gleichermaßen beispielsweise ein ICD (Kardioverter/Defibrillator) implantiert sein. Hierbei handelt es sich um ein elektronisches Gerät, welches Schockimpulse erzeugen kann, beispielsweise um ein lebensbedrohliches Kammerflimmern zu beenden

Ohne entsprechende Gegenmaßnahme besteht bei einer MRT-Untersuchung vor allem für die kleinflächige Elektrodenspitze 5 die Gefahr einer Erwärmung durch die dabei eingesetzten hochfrequenten Wechselfelder. Diese Erwärmung kann gegebenenfalls zu einem Anstieg der Stimulationsreizschwellen, einer Beeinträchtigung der Wahrnehmungsfunktion (Fehl- bzw. Oversensing) und/oder zu einer dauerhaften Schädigung des umliegenden Herzmuskelgewebes führen. In der Folge kann eine Elektrodendislokation und/oder Herzmuskelperforation auftreten. Zudem kann der Fall eintreten, dass der Schrittmacher 3 falsch therapiert bzw. eine Therapie inhibiert wird.

Diese unerwünschten Folgen können durch die in Fig. 2B gezeigte Abschirmvorrichtung 1 vermieden werden. Die Abschirmvorrichtung 1 ist hier beispielsweise in Form einer Weste ausgebildet, die im Bereich des Thorax 7 des Patienten 2 als wickelbares Teil einfach und sicher für den Zeitraum der MRT-Untersuchung anlegbar ist. Die Abschirmvorrichtung 1 deckt den Bereich des implantierten Schrittmachers 3 und der zugehörigen Elektrodenleitung 4 um den Patienten 2 herum vollständig ab. Eine Befestigung der Abschirmvorrichtung 1 am Patienten 2 kann beispielsweise durch einen Klettverschluss erfolgen, was nicht näher dargestellt ist. Vorzugsweise besteht der Klettverschluss zumindest teilweise aus einem elektrisch leitfähigen Material, um auch in diesem Bereich eine vollständige Abschirmung sicherzustellen. Alternativ wäre gleichermaßen möglich, dass die Abschirmvorrichtung beispielsweise als eine Art Bandage oder als aufklebbarer Einmalartikel ausgebildet ist.

Die Abschirmvorrichtung 1 ist so ausgebildet, dass sie einen nennenswerten Anteil der bei der MRT-Untersuchung eingesetzten, hochfrequenten, elektromagnetischen Wechselfelder absorbiert und damit den Schrittmacher 3 und die Elektrodenleitung 4 vor den Wechselfeldern abschirmt. Zu diesem Zweck umfasst die Abschirmvorrichtung 1 ein elektrisch leitfähiges Abschirmmaterial 10, beispielsweise ein elektrisch leitfähiges Polymer oder ein Geflecht aus Metall- oder Kohlefaserfäden, welches die Abschirmvorrichtung 1 zumindest teilweise, insbesondere vollständig überdeckt. Denkbar wäre auch, dass die Abschirmvorrichtung 1 zumindest einen Hohlraum aufweist, der mit einem elektrisch leitfähigen, flüssigen Abschirmmaterial 10 befüllt ist. Diese Variante hat den besonderen Vorteil, dass die elektrische Leitfähigkeit und/oder das Volumen der Flüssigkeit bzw. Abschirmmaterial 10 und/oder deren Art wahlfrei und gezielt auf die Bedürfnisse der Nutzer, d. h. auf die gewünschte Dämpfungswirkung bei bestimmten Scan-Parametern, einstellbar sind. Insbesondere kann die elektrisch leitfähige Flüssigkeit auch durch eine andere, besser oder schlechter leitfähige bzw. elektrisch nicht leitfähige Flüssigkeit ausgetauscht werden. Zudem kann die Abschirmvorrichtung 1 der Kontur der Körperoberfläche des Patienten 2 in einfacher Weise angepasst werden, um einen optimalen Abschirmeffekt zu erreichen. Die Abschirmvorrichtung 1 weist eine relative magnetische Permeabilität von maximal 100, insbesondere maximal 4 auf, insbesondere maximal 1 auf, so dass es für die in der MRT-Untersuchung verwendeten starken statischen Magnetfelder sowie für die dabei eingesetzten magnetischen Gradientenfelder transparent ist.

In besonders vorteilhafter Weise können bei der MRT-Untersuchung die elektromagnetischen Wechselfelder während einer Anregungsphase, in der die hochfrequenten elektromagnetischen Wechselfelder zur resonanten Anregung von Atomkernen erzeugt werden, gedämpft und während einer Auslesephase, in der die angeregten Atomkerne relaxieren und die dabei ausgesandten elektromagnetischen Wechselfelder vom Magnetresonanztomographen zur Bildgebung erfasst werden, zumindest schwächer oder sogar nicht gedämpft werden. Dies kann in einfacher Weise durch eine Änderung der elektrischen Leitfähigkeit und/oder des Volumens des flüssigen Abschirmmaterials 10 erreicht werden. Weiterhin ist es von Vorteil, wenn die Abschirmvorrichtung 1 über eine (nicht dargestellte) Kühleinrichtung zu deren aktiven oder passiven Kühlung verfügt, um den Patientenkomfort zu verbessern und eine übermäßige Erwärmung der Abschirmvorrichtung 1 bei länger dauernden MRT-Untersuchungen zu vermeiden. Eine passive Kühlung kann beispielsweise durch Kühlakkus erreicht werden.

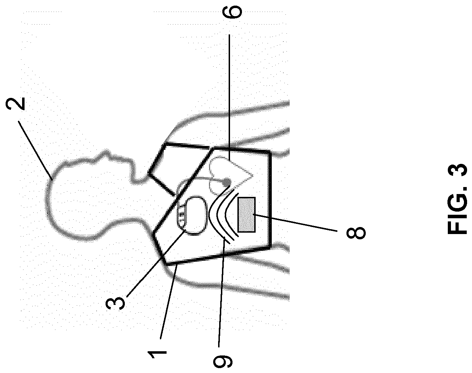

Fig. 3 zeigt eine Variante, bei der die als Weste ausgebildete Abschirmvorrichtung 1 mit einer Kommunikationseinrichtung 8 versehen ist. Die Kommunikationseinrichtung 8 ist in der Lage, ein elektrisches Signal 9 drahtlos an den Schrittmacher 3 zu senden, wobei auf diese Weise die Information übermittelt werden kann, dass die Abschirmvorrichtung 1 verwendet und eine MRT-Untersuchung durchgeführt wird. Der Schrittmacher 3 kann beispielsweise so programmiert sein, dass er daraufhin in einen MRT-Betriebsmodus schaltet, der zur Vermeidung von Fehlfunktionen an die MRT-Untersuchung speziell angepasst ist. Die Kommunikationseinrichtung 8 verfügt zu diesem Zweck über nicht näher dargestellte Mittel zur Erkennung eines statischen Magnetfelds und/oder Gradientenfelds und/oder elektromagnetischen Hochfrequenzfelds, sowie über Mittel zum Erzeugen und Senden der Signale 9, die vorzugsweise eine vom Schrittmacher 3 erkennbare Kodierung aufweisen, um diesen vorzugsweise temporär in den MRT-Betriebsmodus zu versetzen. Die Kommunikationseinrichtung 8 kann die benötigte Energie beispielsweise aus den bei der MRT-Untersuchung eingesetzten Feldern beziehen, wobei sie zu diesem Zweck über nicht näher dargestellte Mittel zur Energiegewinnung und Energiezwischenspeicherung (z. B. Kondensator, Akkumulator) verfügt. Die Aktivität der Kommunikationseinrichtung 8 wird somit ausschließlich durch externe Energie gesteuert, wobei der Schrittmacher 3 bei fehlender Aktivität der Kommunikationseinrichtung 8 die Information erhält, dass die Bildgebung bzw. das Erzeugen hochfrequenter Wechselfelder beendet ist und somit wieder in den normalen Betriebsmodus schalten kann. Alternativ wäre es auch möglich, dass die Kommunikationseinrichtung 8 durch einen eigenen Energiespeicher (Batterie) versorgt wird.

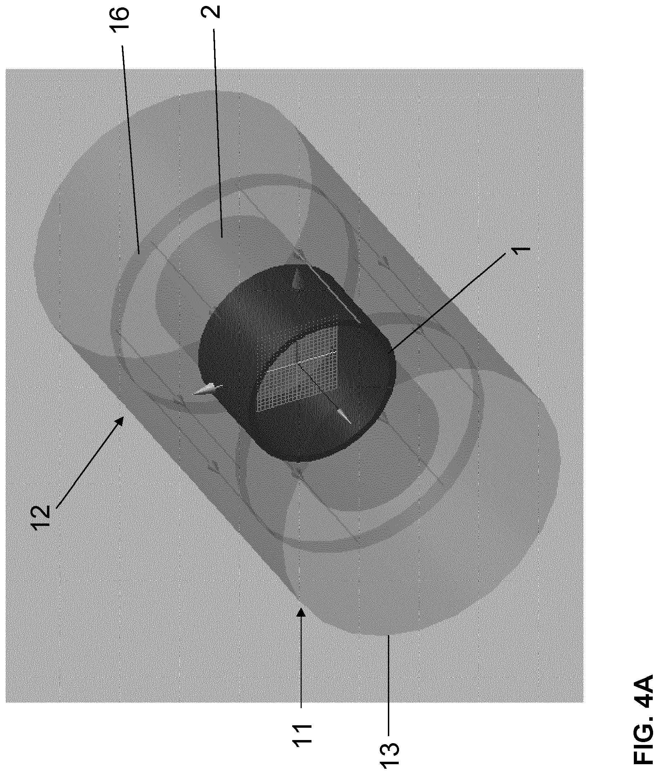

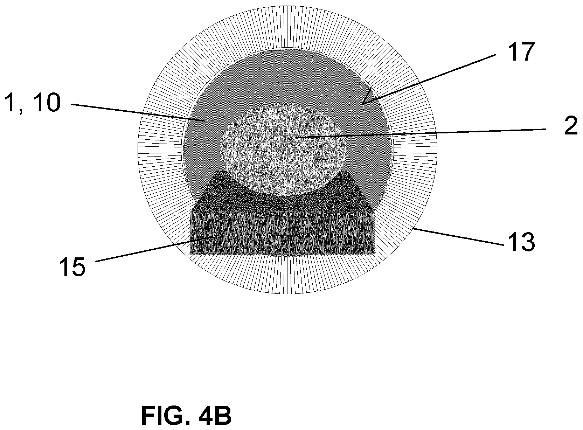

In den Figuren 4A und 4B ist ein weiteres Ausführungsbeispiel der Erfindung in schematischer Weise veranschaulicht. Fig. 4A zeigt eine (transparente) perspektivische Darstellung, Fig. 4B einen Vertikalschnitt durch Fig. 4A. Demnach umfasst eine insgesamt mit der Bezugszahl 11 bezeichnete Abschirmanordnung einen Magnetresonanztomographen 12 mit einer röhrenförmigen Aufnahme bzw. Patientenzylinder 13, in dem ein schematisch dargestellter Patient 2 auf einer Patientenliege 15 zur Untersuchung zu platzieren ist. Mittels eines zylindrischen Hochfrequenzresonators 16 kann für die MRT-Untersuchung ein elektromagnetisches Wechselfeld erzeugt werden. Dabei dient der Patientenzylinder 13 zur Abschirmung der Wechselfelder nach außen hin. Am Körper des Patienten 2 ist eine Abschirmvorrichtung 1 angeordnet, die wie in den zuvor erläuterten Ausführungsbeispielen beispielsweise als Weste zur Abschirmung eines Implantats im thorakalen Bereich ausgebildet sein kann. Wesentlich hierbei ist, dass die Abschirmvorrichtung 1 mit einem beispielsweise flüssigen Abschirmmaterial 10 soweit aufgefüllt ist, dass sie sich einerseits der Körperoberfläche des Patienten 2 anschmiegt und andererseits den Raum 14 bis zur Innenwand 17 des Patientenzylinders 13 hin vollständig ausfüllt. Hierdurch kann ein besonders effektiver Abschirmeffekt erzielt werden. Durch die räumlich versetzbare Patientenliege 15 kann der Patient 2 innerhalb des Patientenzylinders 13 wahlfrei in eine gewünschte Position gebracht werden, beispielsweise zentriert innerhalb der Abschirmvorrichtung 1. Die Abschirmvorrichtung 1 hat eine zylindrische äußere Kontur, die hier beispielsweise koaxial zum Hochfrequenzresonator 16 angeordnet ist, wodurch ein besonders guter Abschirmeffekt erzielt werden kann.

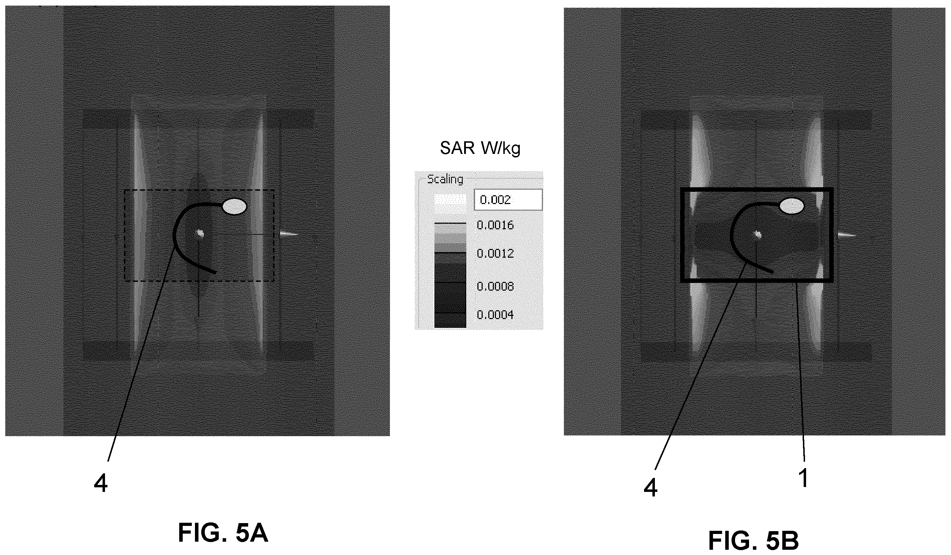

Für eine Situation, wie sie in den Figuren 4A und 4B gezeigt ist, ist in den Figuren 5A und 5B eine Verteilung der im Körper des Patienten 2 im Bereich der Elektrodenleitung 4 bei der MRT-Untersuchung erzeugten Energiedichten (SAR = spezifische Absorptionsrate) in W/kg dargestellt, wobei Figur 5A die Situation ohne Abschirmvorrichtung 1 und Figur 5B die Situation mit Abschirmvorrichtung 1 zeigt. Diese basieren auf einer rechnerischen Simulation, bei der der Patient 2 zentral im Scanner bzw. Patientenzylinder 13 liegt (worst case scenario). Durch die Abbildungen wird klar erkennbar, welche Energiedichten in der unmittelbaren Umgebung der Elektrodenleitung 4 vorliegen und welcher vorteilhafte Effekt dabei durch die Abschirmvorrichtung 1 erreicht werden kann.

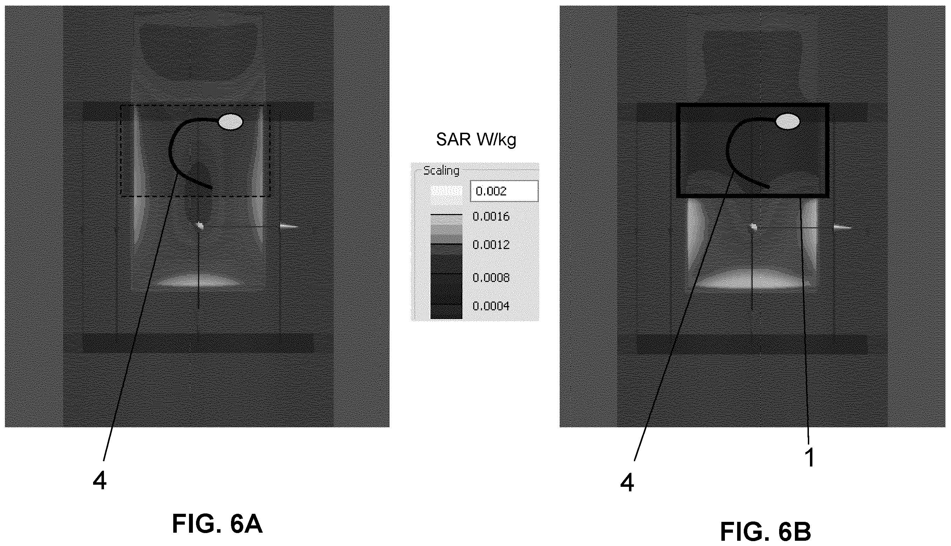

Für eine Situation, wie sie in den Figuren 4A und 4B gezeigt ist, ist in den Figuren 6A und 6B eine Verteilung der im Körper des Patienten 2 im Bereich der Elektrodenleitung 4 bei der MRT-Untersuchung erzeugten Energiedichten (SAR = spezifische Absorptionsrate) in W/kg dargestellt, wobei Figur 6A die Situation ohne Abschirmvorrichtung 1 und Figur 6B die Situation mit Abschirmvorrichtung 1 zeigt. Diese basieren auf einer rechnerischen Simulation, bei der der Patient 2 dezentral im Scanner bzw. Patientenzylinder 13 liegt (Abdomen-Scan). Auch durch diese Abbildungen wird klar erkennbar, welche Energiedichten in der unmittelbaren Umgebung der Elektrodenleitung 4 vorliegen und welcher vorteilhafte Effekt dabei durch die Abschirmvorrichtung 1 erreicht werden kann.

Für eine Situation, wie sie in den Figuren 4A und 4B gezeigt ist, gibt die folgende Tabelle Simulationsergebnisse bezüglich der abschirmenden Wirkung der Abschirmvorrichtung 1 an. Dabei bedeuten:
espr: relative Permittivität des Füllmediums bzw. Abschirmmaterials 10
sigma: Leitfähigkeit in S/m
oben: Körperregion oberhalb des Bereichs der abgeschirmt wird
unten: Körperregion unterhalb des Bereichs der abgeschirmt wird
Mitte: abgeschirmter Bereich

| | Mean SAR (mW/Kg/1W Scanner Leistung) | | |
|---|---|---|---|
| | oben | Mitte | unten |
| Ohne Abschirmvorrichtung | 0.89 | 1.06 | 0.81 |
| Füllung_metallisch | 1.08 | 0.2 | 0.98 |
| Füllung_epsr3_sigma100 | 1.06 | 0.19 | 0.95 |
| Füllung_epsr3_sigma1000 | 1.08 | 0.2 | 0.99 |
| Füllung_epsr80_sigmal | 0.98 | 0.66 | 0.88 |
| Füllung_epsr80_sigma2 | 0.98 | 0.47 | 0.89 |
| Füllung_epsr80_sigma5 | 1 | 0.3 | 0.9 |
| Füllung_epsr80_sigma10 | 1.01 | 0.24 | 0.91 |
| Füllung_epsr80_sigma100 | 1.05 | 0.19 | 0.95 |

Demnach kann die in den Körper des Patienten 2 im Bereich der Abschirmvorrichtung 1 eingebrachte Energie, bezogen auf die ober- bzw. unterhalb der Abschirmvorrichtung 1 eingebrachte Energie, wesentlich reduziert werden, wobei insbesondere eine Reduktion in etwa um einen Faktor 5 ermöglicht ist. Näherungsweise kann die Temperatur der Elektrodenleitung 4 um diesen Faktor reduziert werden.

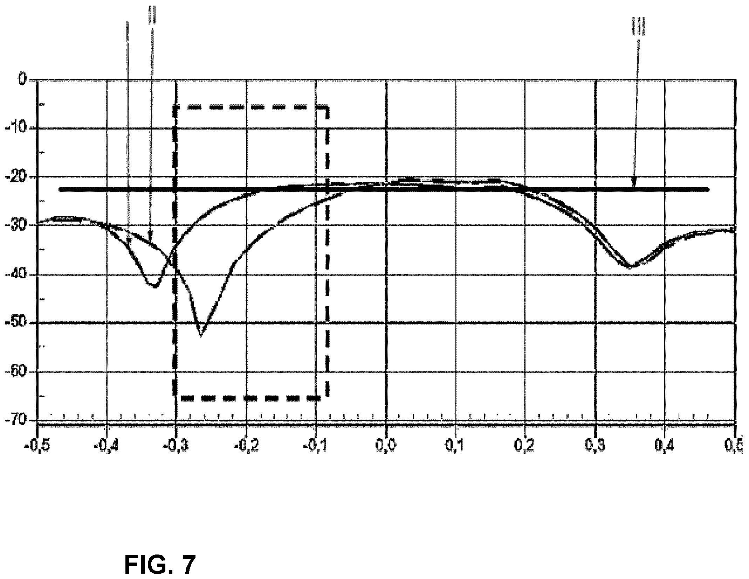

Fig. 7 illustriert die Bildgebungsmöglichkeiten bei Einsatz der erfindungsgemäßen Abschirmvorrichtung bei einer MRT-Untersuchung. Als Abszisse ist der Ort in m, als Ordinate der Feldstärkepegel dB des B1-Felds angegeben. Die Kurve I illustriert eine Situation ohne Abschirmvorrichtung, die Kurve II mit Abschirmvorrichtung. Oberhalb der Kurve III reicht die Feldstärke des B1-Felds für Bildgebung aus. Die gestrichelte Linie gibt den Bereich der Abschirmung an. Demnach bleibt das B1-Feld auch im Falle einer Abschirmung zirkular polarisiert, d. h. die x- und y-Komponenten sind gleich stark, und wird nur lokal gedämpft. Es wird lediglich das Gesichtsfeld (FOV = field of view) etwas eingeschränkt. Hieraus kann geschlossen werden, dass mit Abschirmvorrichtung eine Bildgebung bis 4 mindestens 4 cm vor dem Ende (Randbereich) der Abschirmvorrichtung noch möglich ist.

Wie anhand der Ausführungsbeispiele ausführlich erläutert ist, ermöglicht die vorliegende Erfindung eine einfache, kostengünstige und für den Patienten gefährdungsfreie MRT-Untersuchung, ohne dass spezielle Vorkehrungen am Implantat zu treffen sind. Insbesondere können auch Patienten, die bereits ein Implantat besitzen, einer MRT-Untersuchung unterzogen werden, wobei insbesondere keine besonderen Einstellungen am Magnetresonanztomographen erforderlich sein können. Beispielsweise kann ein ICD auch im Magnetresonanztomographen eine antitachykarde oder antibradykarde Therapie abgeben. Ein besonderer Vorteil besteht in der Möglichkeit auch defekte oder stillgelegte Sonden und lange Stents wie Gefäßprothesen abzuschirmen.

### Bezugszeichenliste

- 1: Abschirmvorrichtung
- 2: Patient
- 3: Schrittmacher
- 4: Elektrodenleitung
- 5: Elektrodenspitze
- 6: Herz
- 7: Thorax
- 8: Kommunikationseinrichtung
- 9: Signal
- 10: Abschirmmaterial
- 11: Abschirmanordnung
- 12: Magnetresonanztomograph
- 13: Patientenzylinder
- 14: Raum
- 15: Patientenliege
- 16: Hochfrequenzresonator
- 17: Innenwand

## Patentansprüche

1. Abschirmvorrichtung (1) zur Abschirmung wenigstens eines zumindest teilweise aus einem metallischen Material bestehenden Implantats (3, 4) im Körper eines Patienten (2) bei einer magnetresonanztomographischen Untersuchung, welche vorübergehend am oder nahe dem Körper des Patienten (2) anbringbar ist und ein Abschirmmaterial (10) umfasst, das geeignet ist, hochfrequente elektromagnetische Wechselfelder zu dämpfen, wobei die Abschirmvorrichtung (1) zur Anpassung an eine Kontur einer Körperoberfläche des Patienten (2) geeignet ausgebildet ist,
**dadurch gekennzeichnet, dass**
die Abschirmvorrichtung (1) mit einem gasförmigen und/oder flüssigen Fluid (10) zur Anpassung an die Kontur der Körperoberfläche des Patienten (2) befüllbar ist.

2. Abschirmvorrichtung (1) nach Anspruch 1, **dadurch gekennzeichnet, dass** sie eine relative magnetische Permeabilität von maximal 100, insbesondere von maximal 4, insbesondere von maximal 1 aufweist.

3. Abschirmvorrichtung (1) nach Anspruch 1 oder 2, **dadurch gekennzeichnet, dass** eine physikalische und/oder chemische Eigenschaft des Abschirmmaterials (10) zur Änderung seines Dämpfungsvermögens zur Dämpfung der elektromagnetischen Wechselfelder veränderbar ist.

4. Abschirmvorrichtung (1) nach einem der Ansprüche 1 bis 3, **dadurch gekennzeichnet, dass** eine Menge des Abschirmmaterials (10) veränderbar ist.

5. Abschirmvorrichtung (1) nach einem der Ansprüche 1 bis 4, **dadurch gekennzeichnet, dass** sie eine Kühleinrichtung zu ihrer Kühlung aufweist.

6. Abschirmvorrichtung (1) nach einem der Ansprüche 1 bis 5, bei welchem die Abschirmvorrichtung mit einer Kommunikationseinrichtung (8) zur Kommunikation mit dem Implantat (3, 4) versehen ist.

7. Abschirmvorrichtung (1) nach einem der Ansprüche 1 bis 6, **dadurch gekennzeichnet, dass** sie eine Markierungseinrichtung zu ihrer Markierung in einer bei der Untersuchung erstellten magnetresonanztomographischen Aufnahme aufweist.

## Claims

1. A shielding apparatus (1) for shielding at least one implant (3, 4), consisting at least partially of a metallic material, in the body of a patient (2) during an examination by magnetic resonance imaging, which apparatus can be attached temporarily on or in the vicinity of the body of the patient (2) and comprises a shielding material (10), which is suitable for attenuating high-frequency alternating electromagnetic fields, wherein the shielding apparatus (1) is suitable for adaptation to a contour of a body surface of the patient (2),
**characterised in that**
the shielding apparatus (1) can be filled with a gaseous and/or liquid fluid (10) for adaptation to the contour of the body surface of the patient (2).

2. The shielding apparatus (1) according to claim 1, **characterised in that** it has a relative magnetic permeability of at most 100, in particular of at most 4, in particular of at most 1.

3. The shielding apparatus (1) according to claim 1 or 2, **characterised in that** a physical and/or chemical property of the shielding material (10) can be varied in order to change the ability thereof to attenuate the alternating electromagnetic fields.

4. The shielding apparatus (1) according to any one of claims 1 to 3, **characterised in that** a quantity of the shielding material (10) can be varied.

5. The shielding apparatus (1) according to any one of claims 1 to 4, **characterised in that** it has a cooling device for cooling said shielding apparatus.

6. The shielding apparatus (1) according to any one of claims 1 to 5, in which the shielding apparatus is provided with a communication device (8) for communication with the implant (3,4).

7. The shielding apparatus (1) according to any one of claims 1 to 6, **characterised in that** it has a marking device for marking said shielding apparatus in a magnetic resonance image created during the examination.

## Revendications

1. Dispositif de blindage (1) pour la protection d'au moins un implant (3, 4) constitué au moins partiellement d'un matériau métallique dans le corps d'un patient (2) lors d'un examen de tomographie par résonance magnétique, lequel peut être appliqué de manière temporaire sur ou près du corps du patient (2) et comprend un matériau de blindage (10) qui est approprié pour amortir des champs alternatifs électromagnétiques de haute fréquence, où le dispositif de blindage est conçu approprié pour un ajustement au contour d'une surface corporelle du patient (2),
**caractérisé en ce que**
le dispositif de blindage (1) peut être rempli avec un fluide (10) sous forme gazeuse et/ou liquide pour l'ajustement au contour de la surface corporelle du patient (2).

2. Dispositif de blindage (1) selon la revendication 1, **caractérisé en ce qu'**il présente une perméabilité magnétique relative d'au maximum 100, notamment d'au maximum 4, en particulier d'au maximum 1.

3. Dispositif de blindage (1) selon la revendication 1 ou 2, **caractérisé en ce qu'**une propriété physique et/ou chimique du matériau de blindage (10) peut être changée pour la modification de sa capacité d'amortissement en vue de l'amortissement des champs alternatifs électromagnétiques.

4. Dispositif de blindage (1) selon l'une des revendications 1 à 3, **caractérisé en ce qu'**une quantité du matériau de blindage (10) peut être changée.

5. Dispositif de blindage (1) selon l'une des revendications 1 à 4, **caractérisé en ce qu'**il présente une unité de refroidissement pour son refroidissement.

6. Dispositif de blindage (1) selon l'une des revendications 1 à 5, chez lequel le dispositif de blindage est muni d'une unité de communication (8) pour la communication avec l'implant (3, 4).

7. Dispositif de blindage (1) selon l'une des revendications 1 à 6, **caractérisé en ce qu'**il présente une unité de marquage pour son marquage dans un enregistrement établi lors de l'examen de tomographie par résonance magnétique.
